# EUROPEAN PATENT SPECIFICATION

(11) **EP 3 488 898 B1**
(45) Date of publication and mention of the grant of the patent: **07.09.2022**
(21) Application number: 18202065.1
(22) Date of filing: 23.10.2018
(51) Int. Cl.: A61N 1/36

(54) **THERAPEUTIC DEVICE FOR TREATING THE HUMAN BODY**
THERAPEUTISCHES GERÄT ZUR BEHANDLUNG DES MENSCHLICHEN KÖRPERS
DISPOSITIF THÉRAPEUTIQUE POUR TRAITEMENT DU CORPS HUMAIN

(30) Priority: 24.11.2017 IT 201700135072
(43) Date of publication of application: 29.05.2019
(73) Proprietor: UNITEKNO S.R.L., 06034 Foligno (PG) (IT)
(72) Inventor: BARTOLINI, Villelmo, 06034 Foligno (PG) (IT)
(74) Representative: Baldi, Claudio

(56) References cited:
- US-A1- 2007 173 908
- US-A1- 2011 125 214
- US-A1- 2013 006 154
- US-A1- 2016 136 424
- US-A1- 2016 303 371

## Description

The present invention relates to a therapeutic device for treating the human body, and particularly to a therapeutic device provided with one or more terminals that are applied to the human body to generate a signal for treating the human body with preset intensity and frequency. Such types of therapeutic devices can be, for example, electromagnetotherapy devices, ultrasound devices, electrostimulators, massaging devices and the like.

Although the following description refers to electromagnetotherapy devices, the invention also relates to similar devices in which parameters are to be adjusted according to the physiognomy of the patients, the body part to be treated and the pathology.

Magnetotherapy devices use low frequency electromagnetic fields, high frequency electromagnetic fields or combined electromagnetic fields for treating pathologies. These devices generate magnetic fields with moderate intensity that favor the vascularization and the regeneration capacity of the human tissues. Therefore, these devices can be used to treat multiple osteoarticular pathologies, such as fractures, various forms of arthrosis, osteoporosis and the like.

These electromagnetotherapy devices are provided with terminals that consist in solenoids. The therapy is typically administered by placing the solenoids in the part to be treated. The solenoids generate an electromagnetic field with frequency and power that correspond to the electrical signal transmitted by a signal generator.

In order to obtain the maximum efficacy, the frequency and intensity of the electromagnetic field must be decided according to the pathology. Given that the intensity of the electromagnetic field decreases when the distance from the solenoid increases, the power of the electrical signal must be varied also according to the body part to be treated and to the patient's physiognomy.

In rehabilitation practices, the correct frequency and the correct power of the electromagnetic field generated by the terminal of the electromagnetotherapy device are set by the physiotherapist, using freely-programmable devices.

Since magnetotherapy requires long daily sessions, devices for domestic use are very popular. In these devices, the problem related with the correct configuration of the magnetic field is solved by providing a long list of therapeutic programs to the patient, who needs to configure the machine according to the program contained in the list.

However, said lists of therapeutic programs do not cover the endless number of possible combinations in terms of intensity and frequency of the magnetic field and are inevitably simplified.

Likewise, the adjustment of parameters about the physiognomy, the body part to be treated and the pathology of the patient is also required when using other devices for treating the human body, which are similar to electromagnetotherapy devices. Therefore the invention also relates to said devices, which are similar to electromagnetotherapy devices.

US2013006154 discloses a device for thermal therapies on mammals, comprising a casing with one or more air chambers that can be inflated with a hot or cold fluid in order to heat or cool the body part to be treated.

The purpose of the present invention is to eliminate the drawbacks of the prior art, by devising a therapeutic device that is capable of generating a signal for treating the human body with the correct frequency and intensity according to the therapy and to the body part to be treated, also according to the physiognomy of the individual patient, without the intervention of a specialist, such as a doctor or a physiotherapist.

These purposes are achieved according to the invention with the characteristics of the independent claim 1.

Advantageous embodiments of the invention appear from the dependent claims.

The therapeutic device of the invention is defined by the independent claim 1.

The advantages of the therapeutic device according to the invention are evident. Such a therapeutic device is configured in such a way that the user only needs to enter his or her physiognomic data, the body part to be treated and the pathology, without having to select the intensity and/or frequency of the treatment signal generated by the terminal of the device, which is of electrical or magnetic or electromagnetic type, such as for example a solenoid, a piezoelectric transducer, an electrode or a vibrator.

According to the data entered by the user and to the lookup tables that are stored in the memory of the device, the therapeutic device identifies the correct intensity and/or frequency of the treatment signal to be generated by the terminal and accordingly controls the signal generator in order to transmit the correct electrical signal to the terminal.

Additional features of the invention will appear manifest from the detailed description below, which refers to merely illustrative, not limiting embodiments, wherein:
Fig. 1 is a block diagram of the therapeutic device of the invention.
Fig. 2 is a picture that is displayed on the display of the device for selecting the body parts to be treated;
Figs. 3A and 3B are two pictures that are displayed on the display of the device for the correct positioning of the terminals of the device, respectively on the patient's chest and arm.

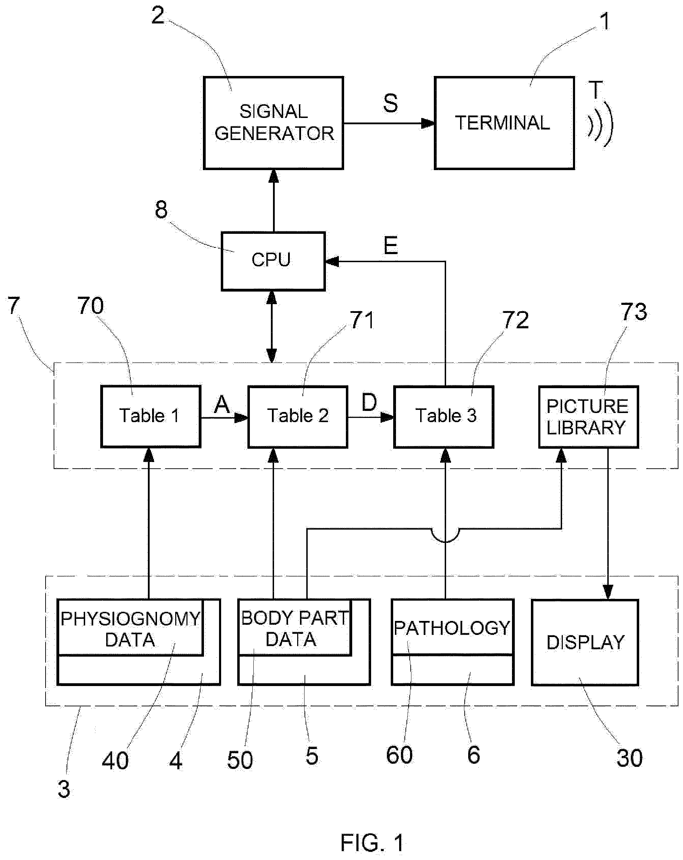

With reference to Fig. 1, the therapeutic device comprises a terminal (1) suitable for being applied in a part of the human body.

The terminal (1) is electrically connected to a signal generator (2) that generates an electrical signal (S) that powers the terminal (1). According to the electrical signal (S) received, the terminal (1) generates a treatment signal (T) that is radiated in the part of the human body to be treated.

The electrical signal (S) is an alternative periodic signal, such as a sinusoid, a square wave, a pulse train or the like. Therefore, the frequency and the intensity of the electrical signal can be adjusted by the signal generator (2). The signal generator can also adjust the duty cycle of the signal, that is the ratio between the pulse duration and the period of the periodic signal.

The terminal (1) is a transducer that transforms the electrical signal (S) into the treatment signal (T) to be radiated in the human body.

If the therapeutic device is an electromagnetotherapy device, the terminal (1) is a solenoid suitable for generating an electromagnetic field.

If the therapeutic device is an ultrasound device, the terminal (1) is a piezoelectric transducer for generating an ultrasound vibration.

If the therapeutic device is an electrostimulator, the terminal (1) is an electrode suitable for generating an electrical signal.

If the therapeutic device is a massaging device, the terminal (1) is a vibrator suitable for generating a vibration.

In any case, the intensity and the frequency of the treatment signal (T) (which can be an electromagnetic field, ultrasound vibrations, an electrical field, non-ultrasound vibrations) are proportional to the intensity and the frequency of the electrical signal (S) generated by the signal generator (2).

The therapeutic device comprises a user interface (3) connected to a CPU (8). The CPU (8) is connected to the signal generator (2) in order to adjust the intensity and frequency of the electrical signal (S) generated by the signal generator.

The user interface (3) comprises a display (30). The display (30) can be a touch screen used by the user to enter data. Alternatively, the user interface (3) can comprise a traditional display with a keyboard and/or a mouse.

The user interface (3) is configured in such a way to comprise:
- first input means (4) for the input of physiognomic data (40) relevant to the patient's physiognomy;
- second input means (5) for the input of body part data (50) relevant to the patient's body parts to be treated and
- third input means (6) for the input of pathological data (60) relevant to the patient's pathology.

A memory (7) contains lookup tables (70, 71, 72) that correlate physiognomies, body parts and pathologies with intensity and/or frequency values of the treatment signal (T) to be obtained,

The first input means (4) can be a window used to enter or select data, which is displayed on the display (30) so that the user can use a mouse, a keyboard or a touch screen to enter or select his or her physiognomic data (40): sex, weight and height.

A first lookup table (70) identifies different types of physiognomy (Physiognomy 1, Physiognomy 2, Physiognomy 3, ...) according to sex (F=female; M=male), weight and height (h).

Following is an example of the first lookup table (70).

**Table 1**

| **Physiognomic values** | **Physiognomy 1** | **Physiognomy 2** | **Physiognomy 3** |
|---|---|---|---|
| F | X | - | - |
| Weight < 45 kg | | | |
| h < 160 cm | | | |
| F, | - | X | - |
| 45 ≤ Weight (kg) ≤ 55 | | | |
| 160 ≤ h (cm) ≤ 165 | | | |
| F, | - | - | X |
| 55 < Weight (kg) ≤ 65 | | | |
| 165 < h (cm) ≤ 170 | | | |
| ... | | | |

For example, the physiognomy type 1 is attributed to a female individual with weight lower than 45Kg and height lower than 160 cm. The physiognomy type 2 is attributed to a female individual with weight comprised between 45Kg and 55 Kg and height comprised between 160 cm and 165 cm, etc. to cover all possible situations.

The physiognomic data (40) entered by the user is compared with the first lookup table (70) to identify a specific physiognomy type (A) of the patient.

The second input means (5) can be a window used to enter or select data, which is displayed on the display (30) so that the user can use a mouse, a keyboard or a touch screen to enter or select the data (50) of the body parts to be treated, such as for example wrist-hands, foot, abdomen, elbow, ankle, arm, thigh, knee, dorsal column, lumbar column, cervical column, pubis, head, chest, hip and shoulder.

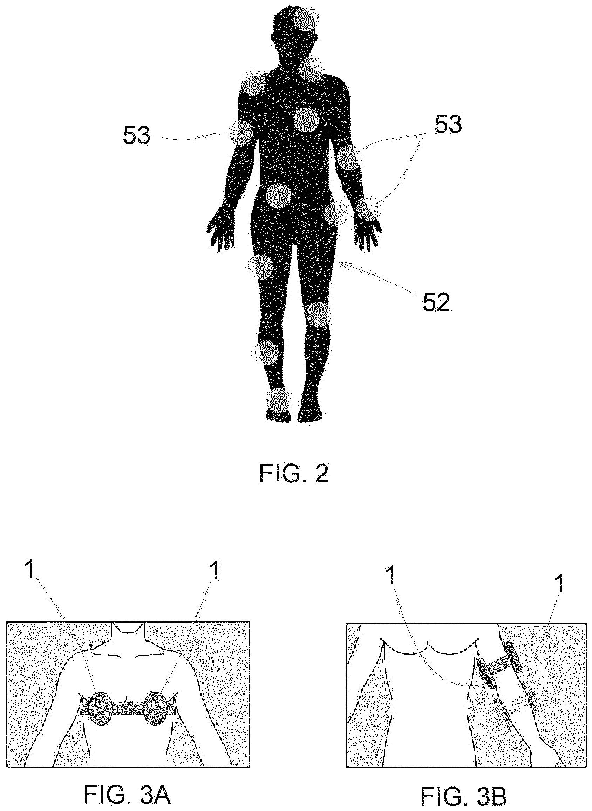

With reference to Fig. 2, advantageously, the second input means (5) comprise a figure of a human body (52) displayed on the display (30); said figure of a human body (52) having a plurality of selectable parts (53) that correspond to the body parts that the user intends to treat; said selectable parts (53) of the figure of the human body can be selected with touch screen or mouse.

A second lookup table (71) identifies various distances from the application point of the terminal to the body part to be treated, according to the different types of patient's physiognomy,

Following is an example of the second lookup table (72).

**Table 2**

| **Body part** | **Typical distance from application point to treated part** | **Variation for physiognomy 1** | **Variation for physiognomy 2** |
|---|---|---|---|
| Wrist - Hands | 1.5 cm | -20% | = |
| Foot | 2.5 cm | -20% | -5% |
| Abdomen | ... | | |
| Elbow | | | |
| Ankle | | | |
| Arm | | | |
| Leg | | | |
| Thigh | | | |
| Knee | | | |
| Dorsal column | | | |
| Lumbar column | | | |
| Cervical column | | | |
| Pubis | | | |
| Head | | | |
| Chest | | | |
| Hip | | | |
| Shoulder | | | |

When the body part to be treated is the foot, if the terminal of the device is positioned on the foot, typically the distance between the terminal and the tissue to be treated is 2.5 cm. However, if the individual belongs to physiognomy 1, then said distance is reduced by 20% and becomes 2 cm. On the contrary, if the individual belongs to physiognomy 2, said distance is reduced by 5% and becomes 2.375 cm.

The specific physiognomy type (A) of the patient identified by comparing the physiognomic data (40) with the first lookup table (70) and the body part data (50) entered by the user are compared in the second lookup table (71) in such a way as to identify a specific distance (D) from the terminal to the part to be treated.

The third input means (6) can be a drop-down menu that is displayed on the display (30), wherein the patient can use a mouse, a keyboard or a touch screen to select the pathology data (60), that is the pathology to be treated. Such pathologies can comprise: fractures, arthrosis, osteoporosis, sprains, and the like.

A third lookup table (72) is stored in the memory (7) for each type of pathology to be treated. Each third lockup table (72) contains intensity and/or frequency values of the treatment signal (T) according to the distance from the terminal (1) to the part to be treated and according to the intensity and frequency or duty cycle of the electrical signal (S) generated by the signal generator.

For illustrative purposes only, following is an example of the third lookup table (72), in the case of a magnetotherapy machine wherein the terminal (1) generates a treatment signal (T) identified by a magnetic field measured in Gauss.

**Table 3**

| **Signal power or duty cycle** | **Distance 0.5 cm** | **Distance 1 cm** | **Distance 1.5 cm** |
|---|---|---|---|
| 100% | 100 Gauss | 95 Gauss | 90 Gauss |
| 90% | 90 Gauss | 85 Gauss | 70 Gauss |
| 80% | 85 Gauss | 80 Gauss | 75 Gauss |
| 70% | ... | | |

In such a case, table 3 shows the values of the magnetic field that are obtained according to the distance from the terminal to the part to be treated and according to the duty cycle of the electrical signal (S) generated by the generator (2). It must be considered that the duty cycle is equal to the pulse duration divided by the period of the waveform; therefore, in any case, the duty cycle depends on the signal frequency.

As shown in table 3, the intensity of the magnetic field emitted by the terminal decreases when the distance from the terminal to the part to be treated increases, and decreases when the duty cycle decreases.

The specific intensity and frequency data (E) identified in the third lookup table (72) is used by the CPU (8) to adjust the intensity and frequency of the signal (S) emitted by the signal generator (2) in such a way as to obtain a correct treatment signal (T) according to the patient's physiognomy, the body part to be treated and the pathology.

The user data (50, 60, 70) can be stored in the memory (7), in such a way as to avoid entering it every time the therapy is repeated.

Furthermore, data of multiple users can be stored in the memory (7) and loaded from a drop-down menu by means of the user interface (3).

Advantageously, the memory (7) contains a library of pictures (73) that show the correct positioning of the terminals of the device according to the body part to be treated.

The CPU (8) is configured in such a way that after the user has entered his or her data (40, 50, 60), according to the body part data (50), the picture of the correct positioning of the terminals on the specific body part is selected from the picture library (73). Such a picture is displayed on the display (30) so that the user knows how to position the terminals.

Fig. 3A shows the picture of the correct positioning of the terminals when the chest is selected by the user. Fig. 3B shows the picture of the correct positioning of the terminals when the arm is selected by the user.

Numerous equivalent variations and modifications can be made to the present embodiments of the invention, which are within the reach of an expert of the field, falling in any case within the scope of the invention.

## Claims

1. Therapeutic device for treating the human body, comprising:
- at least one terminal (1) suitable for being applied to a part of the human body in order to perform a treatment of the human body with intensity and frequency,
- a signal generator (2) configured in such a way as to generate an electrical signal (S) with intensity and frequency proportional to the intensity and frequency of said treatment of the human body,
- a CPU (8) electrically connected to said signal generator to adjust the intensity and frequency of said electrical signal (S) generated by the signal generator,
- a user interface (3) with a display (30), and
- a memory (7),
said user interface (3) is configured in such a way as to comprise:
- first input means (4) for the input of physiognomic data (40) relevant to the patient's physiognomy,
- second input means (5) for the input of body part data (50) relevant to the patient's body parts to be treated, and
- third input means (6) for the input of pathological data (60) relevant to the patient's pathology;
said memory (7) contains lookup tables (70, 71, 72) that correlate physiognomies, body parts and pathologies with intensity and/or frequency-of the treatment to be obtained,
and said CPU (8) is configured in such a way to correlate said physiognomic data (40), body part data (50) and pathology data (60) entered by the patient with said lookup tables (70, 71, 72) stored in the memory in order to find a correct intensity and/or frequency of the treatment and suitably control said signal generator (2) to obtain said correct intensity and/or frequency of the treatment,
**characterized in that**
said terminal (1) is of electrical or magnetic or electromagnetic type,
said signal generator (2) is electrically connected to the terminal (1),
said terminal (1) is a transducer that transforms the electrical signal (S) from the signal generator (2) into a treatment signal (T) to be radiated in the human body; wherein the treatment signal (T) is an electromagnetic field, ultrasound vibrations, an electrical field or non-ultrasound vibrations, having intensity and frequency proportional to the intensity and the frequency of the electrical signal (S) from the signal generator.

2. The therapeutic device of claim 1, wherein
said physiognomic data (40) entered by the patient is: sex, weight and height and
said lookup tables comprise a first lookup table (70) that identifies different types of physiognomy according to sex, weight and height.

3. The therapeutic device of claim 2, wherein
said body part data (50) comprise: wrist-hands, foot, abdomen, elbow ankle, arm, leg, thigh, knee, dorsal column, lumbar column, cervical column, pubes, head, thorax, hip and shoulder and
said lookup tables comprise a second lookup table (71) that identifies various distances from the application point of the terminal to the body part to be treated, according to different types of patient's physiognomy.

4. The therapeutic device of claim 3, wherein
said pathology data (60) comprises: fractures, arthrosis, osteoporosis, sprains;
said lookup tables comprise a third lookup table (72) associated with each pathology; each third lookup table (72) contains intensity and/or values of the treatment signal (T) according to the distance of the terminal (1) from the part to be treated and according to the intensity and frequency or duty cycle of the electrical signal (S) generated by the signal generator.

5. The therapeutic device of any one of the preceding claims, comprising a picture library (73) stored in said memory (7); said picture library comprising pictures relative to a correct positioning of the terminals of the device according to the body part to be treated;
said CPU (8) being configured in such a way that when the user has entered his or her physiognomic data, body part data and pathology data (40, 50, 60), the picture showing the correct positioning of the terminals on the body part selected by the user is selected from the picture library (73) according to the body part data (50), and said picture is displayed on the display (30) in such a way that the user can have information on the positioning of the terminals.

6. The therapeutic device of any one of the preceding claims, wherein the second input means (5) comprise a figure of a human body (52) displayed on the display (30); said figure of a human body (52) having a plurality of selectable parts (53) that correspond to the body parts to be treated by the user; said selectable parts (53) of the figure of the human body being selectable with a touch screen or a mouse.

## Patentansprüche

1. Therapeutische Vorrichtung zur Behandlung des menschlichen Körpers, umfassend:
- mindestens ein Endteil (1), das dazu bestimmt ist, an einem Teil des menschlichen Körpers angewendet zu werden, um eine Behandlung des menschlichen Körpers mit einer Intensität und Frequenz auszuführen,
- einen Signalgenerator (2), der konfiguriert ist, um ein elektrisches Signal (S) mit einer Intensität und Frequenz zu emittieren, die proportional zur Intensität und Frequenz der Behandlung des menschlichen Körpers sind,
- eine Steuereinheit (8), die elektrisch mit dem Signalgenerator verbunden ist, um die Intensität und Frequenz des vom Signalgenerator emittierten elektrischen Signals (S) zu regeln,
- eine Benutzerschnittstelle (3) mit einem Display (30), und
- einen Speicher (7),
die Benutzerschnittstelle (3) ist so konfiguriert, dass sie umfasst:
- erste Eingabemittel (4) für die Eingabe physiognomischer Daten (40) bezüglich der Physiognomie des Patienten,
- zweite Eingabemittel (5) für die Eingabe von Körperteildaten (50) bezüglich der Körperteile des zu behandelnden Patienten, und
- dritte Eingabemittel (6) für die Eingabe pathologischer Daten (60) bezüglich der Pathologie des Patienten;
der Speicher (7) enthält Korrelationstabellen (lookup tables) (70, 71, 72), die Physiognomien, Körperteile und Pathologien mit Intensität und Frequenz der zu erhaltenden Behandlung in Korrelation bringen,
die Steuereinheit (8) ist so konfiguriert, dass sie die von dem Patienten eingegebenen physiognomischen Daten (40), Körperteildaten (50) und pathologischen Daten (60) mit den in dem Speicher gespeicherten Korrelationstabellen (70, 71, 72) korreliert, um eine korrekte Intensität und/oder Frequenz der Behandlung zu finden und den Signalgenerator (2) so zu steuern, dass die korrekte Intensität und/oder Frequenz der Behandlung erhalten wird,
**dadurch gekennzeichnet, dass**
das Endteil (1) vom elektrischen oder magnetischen oder elektromagnetischen Typ ist,
der Signalgenerator (2) elektrisch an das Endteil (1) angeschlossen ist,
das Endteil (1) ein Wandler ist, der das von dem Signalgenerator (2) stammende elektrische Signal (S) in ein in dem menschlichen Körper auszustrahlendes Behandlungssignal (T) umwandelt; wobei das Behandlungssignal (T) ein elektromagnetisches Feld, Ultraschallschwingungen, ein elektrisches Feld oder Nicht-Ultraschallschwingungen mit einer Intensität und Frequenz ist, die proportional zur Intensität und Frequenz des vom Signalgenerator stammenden elektrischen Signals (S) sind.

2. Therapeutische Vorrichtung nach Anspruch 1, wobei
die vom Patienten eingegebenen physiognomischen Daten (40) Geschlecht, Gewicht und Größe sind und
die Korrelationstabellen eine erste Korrelationstabelle (70) umfassen, die verschiedene physiognomische Typen entsprechend dem Geschlecht, dem Gewicht und der Größe identifizieren.

3. Therapeutische Vorrichtung nach Anspruch 2, wobei
die Körperteildaten (50) umfassen: Handgelenk-Hände, Fuß, Bauch, Ellbogen, Knöchel, Arm, Bein, Oberschenkel, Knie, Rückenwirbelsäule, Lendenwirbelsäule, Halswirbelsäule, Schambein, Kopf, Brustkorb, Hüfte und Schulter und
die Korrelationstabellen eine zweite Korrelationstabelle (71) umfassen, die verschiedene Abstände vom Anwendungspunkt des Endteils zu dem zu behandelnden Körperteil, entsprechend den verschiedenen Patientenphysiognomietypen identifiziert.

4. Therapeutische Vorrichtung nach Anspruch 3, wobei
die pathologischen Daten (60) umfassen: Brüche, Arthrose, Osteoporose, Verstauchungen;
die Korrelationstabellen eine dritte Korrelationstabelle (72) umfassen, die jeder Pathologie zugeordnet ist; jede dritte Korrelationstabelle (72) enthält Intensitäts- und/oder Frequenzwerte des Behandlungssignals (T) entsprechend dem Abstand des Endteils (1) von dem zu behandelnden Körperteil und basierend auf der Intensität und Frequenz oder des Tastgrads des vom Signalgenerator erzeugten elektrischen Signals (S).

5. Therapeutische Vorrichtung nach einem der vorstehenden Ansprüche, umfassend ein in dem Speicher (7) gespeichertes Bildarchiv (73); wobei das Bildarchiv Bilder bezüglich einer korrekten Positionierung des Endteils der Vorrichtung entsprechend dem zu behandelnden Körperteil umfasst;
wobei die Steuereinheit (8) so konfiguriert ist, dass wenn der/die BenutzerIn seine/ihre physiognomischen Daten, Körperteildaten und pathologischen Daten (40, 50, 60) eingegeben hat, aus dem Bildarchiv (73) das Bild mit der korrekten Positionierung der Endteile auf dem von dem/der BenutzerIn ausgewählten Körperteil (50) ausgewählt wird und das Bild auf dem Display (30) so angezeigt wird, dass der/die BenutzerIn Informationen darüber erhalten kann, wie die Endteile zu positionieren sind.

6. Therapeutische Vorrichtung nach einem der vorstehenden Ansprüche, wobei die zweiten Eingabemittel (5) eine Figur eines menschlichen Körpers (52) umfassen, die auf dem Display (30) angezeigt wird; wobei die Figur eines menschlichen Körpers (52) eine Mehrzahl von auswählbaren Teilen (53) aufweist, die den Körperteilen entsprechen, die der/die BenutzerIn behandeln möchte; wobei die auswählbaren Teile (53) der Figur des menschlichen Körpers mittels Touchscreen oder Maus auswählbar sind.

## Revendications

1. Dispositif thérapeutique pour le traitement du corps humain, comprenant :
- au moins un terminal (1) destiné à être appliqué sur une partie du corps humain, pour effectuer un traitement du corps humain avec intensité et fréquence,
- un générateur de signal (2) configuré de manière à émettre un signal électrique (S) ayant une intensité et une fréquence proportionnelles à l'intensité et fréquence du dit traitement du corps humain,
- une unité de commande électronique (8) branchée électriquement au dit générateur de signal pour régler l'intensité et la fréquence du dit signal électrique (S) émis par le générateur de signal,
- une interface utilisateur (3) équipée d'afficheur (30), et
- une mémoire (7),
ladite interface utilisateur (3) étant configurée de manière à comprendre :
- des premiers moyens d'entrée (4) pour la saisie des données de physionomie (40) relatives à la physionomie du patient,
- des deuxièmes moyens d'entrée (5) pour la saisie de données de parties du corps (50) relatives aux parties du corps du patient à soumettre au traitement, et
- des troisièmes moyens d'entrée (6) pour la saisie des données de pathologie (60) relatives à la pathologie du patient ;
ladite mémoire (7) contient des tables de consultation (lookup tables) (70, 71, 72) qui mettent en corrélation des physionomies, des parties du corps et des pathologies avec l'intensité et/ou la fréquence du traitement que l'on souhaite obtenir,
et ladite unité de commande électronique (8) est configurée de manière à mettre en corrélation lesdites données de physionomie (40), données de parties du corps (50) et données de pathologie (60) saisies du patient avec lesdites tables de consultation (70, 71, 72) enregistrées dans la mémoire pour trouver une intensité et/ou une fréquence correctes du traitement et commander ledit générateur de signal (2) afin d'obtenir ladite intensité et/ou fréquence correctes du traitement,
**caractérisé en ce que**
ledit terminal (1) est du type électrique ou magnétique ou électromagnétique,
ledit générateur de signal (2) est électriquement branché au terminal (1),
ledit terminal (1) est un transducteur qui transforme le signal électrique (S) en provenance du générateur de signal (2) en un signal de traitement (T) à irradier dans le corps humain ; où le signal de traitement (T) est un champ électromagnétique, des vibrations ultrasoniques, un champ électrique ou des vibrations non ultrasoniques ayant intensité et fréquence proportionnelles à l'intensité et à la fréquence du signal électrique (S) en provenance du générateur de signal.

2. Dispositif thérapeutique selon la revendication 1, où
lesdites données de physionomie (40) saisies du patient sont : sexe, poids et hauteur et,
lesdites tables de consultation comprennent une première table de consultation (70) qui identifie des types différents de physionomie sur la base du sexe, du poids et de la hauteur.

3. Dispositif thérapeutique selon la revendication 2, où
lesdites données des parties du corps (50) comprennent : poignet-mains, pied, abdomen, coude, cheville, bras, jambe, cuisse, genou, colonne dorsale, colonne lombaire, colonne cervicale, pubis, tête, thorax, hanche et épaule et,
lesdites tables de consultation comprennent une deuxième table de consultation (71) qui identifie des distances diverses à partir du point d'application du terminal à la partie du corps à traiter, en fonction des différents types de physionomie du patient.

4. Dispositif thérapeutique selon la revendication 3, où
lesdites données de pathologie (60) comprennent : des fractures, des arthroses, des ostéoporoses, des distorsions ;
lesdites tables de consultation comprennent une troisième table de consultation (72) associée à chaque pathologie ; chaque troisième table de consultation (72) contient des valeurs d'intensité et/ou de fréquence du signal de traitement (T) en fonction de la distance du terminal (1) de la partie à traiter et en fonction de l'intensité et de la fréquence ou du cycle de fonctionnement du signal électrique (S) généré par le générateur de signal.

5. Dispositif thérapeutique selon l'une quelconque des revendications précédentes, comprenant une librairie d'images (73) enregistrées dans ladite mémoire (7) ; ladite librairie d'images comprenant des images relatives au positionnement correct des terminaux du dispositif, en fonction de la partie du corps à traiter ;
ladite unité de commande électronique (8) étant configurée de manière qu'une fois que l'utilisateur a saisi ses propres données de physionomie, de parties du corps et de pathologie (40, 50, 60), en fonction des données des parties du corps (50), de la librairie d'images (73) est sélectionnée l'image de positionnement correct des terminaux sur la partie du corps sélectionnée par l'utilisateur et ladite image est affichée sur l'afficheur (30), afin que l'utilisateur puisse savoir comment positionner les terminaux.

6. Dispositif thérapeutique selon l'une quelconque des revendications précédentes, où les deuxièmes moyens de saisie (5) comprennent une figure d'un corps humain (52) affichée sur l'afficheur (30) ; ladite figure d'un corps humain (52) ayant une pluralité de parties sélectionnables (53) correspondantes aux parties du corps que l'utilisateur souhaite traiter ; lesdites parties sélectionnables (53) de la figure du corps humain étant sélectionnables moyennant un écran tactile ou une souris.
